# EUROPEAN PATENT APPLICATION

(11) **EP 4 535 368 A1**
(43) Date of publication of application: **09.04.2025**
(21) Application number: 23201116.3
(22) Date of filing: 02.10.2023
(51) Int. Cl.: G16H 20/00, G16H 50/20

(54) **SELECTING PATIENTS FOR THERAPY**

(71) Applicant: Bayer Aktiengesellschaft, 51373 Leverkusen (DE)
(72) Inventor: Toki, Dale, Mississauga, L4W 5R6 (CA)
(74) Representative: BIP Patents

(57) **Abstract**

Systems, methods, and computer programs disclosed herein relate to the selection of patients for therapy.

## Description

### FIELD OF THE DISCLOSURE

Systems, methods, and computer programs disclosed herein relate to the selection of patients for therapy.

### BACKGROUND

In recent years, medicine has made remarkable advances that have significantly improved medical care and extended people's life expectancy. Breakthroughs in medical research and technology have revolutionized diagnosis, treatment, and patient care.

However, this progress has come at a price, as the cost of medical treatments continues to rise. While these developments promise better health and quality of life, they also pose affordability and accessibility challenges. In this complex environment, it is critical to strike a balance between honoring medical achievements and managing the growing financial burden of health care to ensure that all people have access to the benefits of modern medicine. This delicate balance between medical progress and escalating healthcare costs is a key challenge for societies worldwide.

Personalized medicine is an example of rising costs. Today's medicine is increasingly focused on the individual needs of patients. Whereas in the past patients were predominantly treated with the same methods, today personalized medicine is becoming increasingly prevalent.

Personalized medicine makes it possible to take a "one size fits all" approach to diagnostics, drug therapy, and prevention and turn it into an individualized approach.

Knowing a patient's individual genetic profile, for example, can help physicians select the right drug or therapy and administer it at the right dose or regimen.

Treatment approaches that directly act on a patient's genetic makeup to cure or alleviate a disease are also playing an increasingly important role.

However, personalized medicine usually involves higher costs. Gene therapy in particular is still so expensive today that not all people can afford it if they had to pay for it directly.

However, medical care should not be reserved for people who can afford it. Of course, this applies not only to personalized medicine but also to other therapies that are comparatively expensive.

Rare diseases are another example. A rare disease is one that affects only a small percentage of the population. Its rarity often means that there is no market large enough to gamer support and resources for treatment discovery unless the government grants economically advantageous terms for the development and sale of such treatments. There are thousands of rare diseases. To date, six to seven thousand rare diseases have been discovered and new diseases are regularly described in medical literature. So even if the individual disease is rare, the large number of rare diseases can affect many people. So, there is a need to develop therapies for rare diseases. But here, too, there is the question of funding. Treating a patient suffering from a rare disease can be costly.

### SUMMARY

These and further problems are solved by the subject matter of the independent claims of the present disclosure. Preferred embodiments are defined in the dependent claims, the description, and the drawings.

In a first aspect, the present disclosure relates to a computer-implemented method comprising:
- receiving a request for the issuance of a convertible bond,
   ∘ wherein the convertible bond comprises a patient's right to receive a therapy for a disease if conversion criteria are met,
   ∘ wherein the request comprises initial patient data,
- determining whether issue criteria for issuance of the convertible bond to the patient are met based on the initial patient data,
- in the event that the issue criteria are met: determining the conditions under which the convertible bond is issued based on the initial patient data and/or based on data on convertible bonds already issued to other patients and/or based on data from other patients who suffered from the disease,
- outputting the conditions under which the convertible is issued.

In another aspect, the present disclosure provides a computer system comprising:
a processor; and
a memory storing an application program configured to perform, when executed by the processor, an operation, the operation comprising:
   - receiving a request for the issuance of a convertible bond,
      ∘ wherein the convertible bond comprises a patient's right to receive a therapy for a disease if conversion criteria are met,
      ∘ wherein the request comprises initial patient data,
   - determining whether issue criteria for issuance of the convertible bond to the patient are met based on the initial patient data,
   - in the event that the issue criteria are met: determining the conditions under which the convertible bond is issued based on the initial patient data and/or based on data on convertible bonds already issued to other patients and/or based on data from other patients who suffered from the disease,
   - outputting the conditions under which the convertible is issued.

In another aspect, the present disclosure provides a non-transitory computer readable storage medium having stored thereon software instructions that, when executed by a processor of a computer system, cause the computer system to execute the following steps:
- receiving a request for the issuance of a convertible bond,
   ∘ wherein the convertible bond comprises a patient's right to receive a therapy for a disease if conversion criteria are met,
   ∘ wherein the request comprises initial patient data,
- determining whether issue criteria for issuance of the convertible bond to the patient are met based on the initial patient data,
- in the event that the issue criteria are met: determining the conditions under which the convertible bond is issued based on the initial patient data and/or based on data on convertible bonds already issued to other patients and/or based on data from other patients who suffered from the disease,
- outputting the conditions under which the convertible is issued.

### BRIEF DESCRIPTION OF THE DRAWINGS

Fig. 1 schematically shows an example of an operating model for issuing convertible bonds and converting them into therapies.
Fig. 2 show schematically and by way of example a process of training a machine learning model.
Fig. 3 shows the use of the trained machine learning model to determine a probability value for a patient.
Fig. 4 shows schematically an embodiment of the computer-implemented method of the present disclosure in form of a flow chart.
Fig. 5 illustrates a computer system according to some example implementations of the present disclosure in more detail.

### DETAILED DESCRIPTION

The invention will be more particularly elucidated below without distinguishing between the aspects of the invention (method, computer system, computer-readable storage medium). On the contrary, the following elucidations are intended to apply analogously to all the aspects of the invention, irrespective of in which context (method, computer system, computer-readable storage medium) they occur.

If steps are stated in an order in the present description or in the claims, this does not necessarily mean that the invention is restricted to the stated order. On the contrary, it is conceivable that the steps can also be executed in a different order or else in parallel to one another, unless one step builds upon another step, this absolutely requiring that the building step be executed subsequently (this being, however, clear in the individual case). The stated orders are thus preferred embodiments of the present disclosure.

As used herein, the articles "a" and "an" are intended to include one or more items and may be used interchangeably with "one or more" and "at least one." As used in the specification and the claims, the singular form of "a", "an", and "the" include plural referents, unless the context clearly dictates otherwise. Where only one item is intended, the term "one" or similar language is used. Also, as used herein, the terms "has", "have", "having", or the like are intended to be open-ended terms. Further, the phrase "based on" is intended to mean "based at least partially on" unless explicitly stated otherwise. Further, the phrase "based on" may mean "in response to" and be indicative of a condition for automatically triggering a specified operation of an electronic device (e.g., a controller, a processor, a computing device, etc.) as appropriately referred to herein.

Some implementations of the present disclosure will be described more fully hereinafter with reference to the accompanying drawings, in which some, but not all implementations of the disclosure are shown. Indeed, various implementations of the disclosure may be embodied in many different forms and should not be construed as limited to the implementations set forth herein; rather, these example implementations are provided so that this disclosure will be thorough and complete, and will fully convey the scope of the disclosure to those skilled in the art.

The present disclosure describes means by which patients can access therapy for a disease.

A "patient" is a living being, preferably a mammal, most preferably a human being.

A "disease" is a disordered or incorrectly functioning organ, part, structure, or system of the body of a patient resulting from the effect of genetic and/or developmental errors and/or deviations, infection, poisons, nutritional deficiency or imbalance, toxicity, or unfavorable environmental factors. The term "disease" is to be understood broadly, it can also have the meaning "physical and/or mental condition".

A "therapy" or medical treatment is the attempted remediation of a health problem, usually following a medical diagnosis. A therapy may be an action taken to cure a disease and/or to alleviate the symptoms of a disease and/or to prevent the onset of a disease and/or to prevent the deterioration of a physical and/or mental condition of a patient.

A therapy may include one or more actions taken on and/or with a patient's body or part thereof. Such an action may be the administration of a drug and/or treatment with electromagnetic radiation and/or treatment with particle beams and/or any other measure or a combination of measures.

In an embodiment of the present disclosure, the therapy is an individualized therapy adapted to the individual needs of a patient. For example, the therapy can be a component of personalized medicine.

In an embodiment of the present disclosure, the therapy comprises gene therapy. "Gene therapy" is a medical technology which aims to produce a therapeutic effect through the manipulation of gene expression or through altering the biological properties of living cells.

In an embodiment of the present disclosure, the therapy comprises cell therapy. "Cell therapy" is a therapy in which viable cells are injected, grafted or implanted into a patient in order to effectuate a medicinal effect, for example, by transplanting T-cells capable of fighting cancer cells via cell-mediated immunity in the course of immunotherapy, or grafting stem cells to regenerate diseased tissues.

In an embodiment of the present disclosure, the therapy is a therapy for treating a rare disease. For example, treatment of such a disease may involve administration of an orphan drug.

Patients access therapy through convertible bonds. A "convertible bond", as the term is used in this disclosure, is part of a financing model for a therapy. A "convertible bond" comprises a patient's right to access a therapy at a time in the future. In other words, a convertible bond can be converted into a therapy. A convertible bond is personal and not transferable to other persons.

The therapy into which the convertible bond may be converted is also referred to in this disclosure as "covered therapy". The disease that the covered therapy is intended to treat is also referred to in this disclosure as the "covered disease".

It is possible that a convertible bond covers multiple therapies (i.e., more than one therapy). For simplicity, this disclosure assumes that a convertible bond covers only one therapy (the covered therapy). However, this is not to be understood as a limitation. This applies analogously to the covered disease: it is possible for a convertible bond to cover more than one disease; for simplicity and without wishing to limit the invention, this disclosure assumes that a convertible bond covers only one disease (the covered disease).

Even if patients do not have a disease, they are referred to as "patients" in this description. The term "patient" therefore does not mean that the patient has a disease.

Typically, at the time a patient receives a convertible bond, it is not even known if the covered disease will even develop in the patient. The convertible bond can therefore also be understood as an insurance policy that the patient can fall back on if the covered disease breaks out.

It is possible to sell different versions of convertible bonds: e.g., bonds that cover only the co-pays, bonds offered under employee-sponsored insurance plans, and/or bonds sold to new parents for their babies. Other/further scenarios are conceivable.

In one embodiment, the convertible bond is granted for a predefined period, also referred to as "conversion period" in this disclosure. This means that a patient can only convert the convertible bond into therapy within this conversion period; after the conversion period has expired, the patient can no longer exercise his or her right.

For example, the conversion period can be one year, or two years, or three years, or four years, or five years, or more than five years. Preferably, the conversion period is at least one year and at most ten years, preferably less than ten years.

The conversion period is also not limited to whole years; it can also be a number of days, weeks and/or months.

In a first step, a request for the issuance of a convertible bond is received.

By "receive" is meant to accept, retrieve, or obtain one or more of information, data, executable code, or any result from executing an executable code.

The request is usually made by the patient himself/herself. The request is for a distributor of convertible bonds and may include a request to issue a convertible bond for the patient.

The request may have been transmitted via e-mail, or a graphical interface user interface over the Internet. However, the request may also be received in writing by the convertible bond distributor or therapy provider. A user of the computer system of the present disclosure may enter such request into the computer system.

The distributor of convertible bonds may be the company that also provides the therapy into which the convertible bond may be converted, or a company that distributes convertible bonds on behalf of the therapy provider, or another company (each such party is also referred to in this disclosure as a "convertible bond issuer").

Before a convertible bond is issued, an assessment is made as to whether and/or under what conditions the convertible bond can/will be issued to the patient. The assessment is based (at least partially) on initial patient data.

Initial patient data includes patient health data at the time of the patient's request for the issuance of a convertible bond. Initial patient data may include, e.g.,: age, sex, body size (height), body weight, body mass index, blood group, blood values, blood pressure values, resting heart rate, heart rate variability, an electrocardiogram, sugar concentration in urine and/or blood (e.g., hemoglobin A1C) and/or other values from urine/blood tests, body temperature, impedance (e.g., thoracic impedance), lifestyle information about the life of the patient, such as consumption of alcohol, smoking, and/or exercise and/or the patient's diet, medical intervention parameters such as regular medication, occasional medication, or other previous or current medical interventions and/or other information about the patient's previous and/or current treatments and/or reported health conditions and/or combinations thereof.

Initial patient data may include the results of genetic testing on the patient and/or other results of other laboratory tests.

Initial patient data may include medical images such as x-rays and/or magnetic resonance imaging images and/or computed tomography images and/or ultrasound images and/or positron emission tomography images.

Initial patient data may include a completed questionnaire in which the patient has provided answers to questions posed to him/her, for example, online via a graphical user interface. An answer may also be a selection of one or more characteristics from a list and/or the ticking of a box.

Based on the initial patient data (and optionally further data), the computer system of the present disclosure automatically examines whether predefined criteria are met.

The predefined criteria determine whether a convertible bond is issued to the patient at all (issue criteria). The issue criteria are usually set by the distributor of the convertible bonds and/or the provider of the therapy. The issue criteria can also be set by an independent third party such as a health insurance company or a health authority. The definition and/or specification of the issue criteria may be based, for example, on medical and/or economic and/or social and/or other considerations.

The examination whether the issue criteria are met usually involves analysing whether the patient is already suffering from the covered disease and/or showing signs of the covered disease. Typically, a patient who already suffers from the covered disease and/or shows signs of the covered disease is excluded from convertible bonds for therapies against the covered disease. Typically, convertible bonds are intended for patients who may in principle have the covered disease in the future, but do not currently show signs of the covered disease. Acute action is needed for patients already showing signs of the covered disease, and convertible bonds are usually not the right tool for such patients.

The analysis of the initial patient data is done automatically, i.e., without human intervention.

Determining whether a patient is eligible for a convertible bond may include analysis of health information about the patient's dependents, such as health information about the patient's parents and/or grandparents and/or siblings.

Patients whose relatives already have or had the covered disease usually have a higher risk of developing the covered disease, especially in the case of genetic diseases, than patients whose relatives do not have or had the covered disease. Thus, if the covered disease is a genetic disease, a patient may be denied a convertible bond if the covered disease has already appeared in relatives. However, it is also possible that such a patient will have to pay a higher price for a convertible bond and/or that the term (e.g., conversion period) of the convertible bond will be shorter.

If the analysis of the initial patient data and, if applicable, further data has shown that a convertible bond can be issued for the patient (if the issue criteria are met), a determination is made of the conditions under which the convertible bond is issued. The conditions are determined on the basis of the initial patient data, optionally further data on the patient and/or his/her relatives, and/or on the basis of data on convertible bonds already issued to other patients and/or on the basis of data from patients who suffered from the covered disease.

Conditions may include, for example, a price that the patient must pay to obtain a convertible bond. Conditions may include, for example, the conversion period within which the convertible bond can be converted into therapy. Conditions may include, for example, the conversion criteria that must be met in order for the patient to convert the convertible bond into therapy.

The patient may be refunded a portion of the price paid for the convertible bond at the end of the conversion period; the conditions may provide for such a refund amount. The patient may invest in the convertible bond and receive interest on it; the terms may include the amount of such interest. Typically, this interest is lower than the interest on other investments in the market because a convertible bond includes the benefit of conversion to therapy.

The conditions (e.g., price, conversion period, interest rate, and/or other) for the issuance of a convertible bond may be determined/set based on the frequency of the covered disease, the number of convertible bonds already sold and/or converted for the covered disease, the patient's health status and/or lifestyle, the cost of therapy, and/or other/further parameters.

Conditions can be output, such as shown on a display, output to a printer, stored in a data storage device, and/or transmitted to a separate computer system (e.g., the patient's computer system).

In analysing whether a patient is eligible for a convertible bond and/or in determining the conditions under which such a convertible bond is issued to the patient, the likelihood that the patient will develop the covered disease may also be determined.

For example, a probability value may be determined that indicates how likely it is that the patient will develop the covered disease at all and/or within a predefined period (e.g., within the conversion period). The probability value can be a number between 0 and 1, or a percentage between 0 and 100, or any other value.

Typically, the probability value is positively correlated with the likelihood, i.e., the higher the probability that the patient will develop the covered disease, the higher the probability value.

The probability value can also indicate a period of time within which the patient will or will not develop the covered disease or will develop the covered disease with a pre-defined probability.

The probability value may also indicate a severity of the disease that is expected for the patient.

The probability value may be determined based on the initial patient data and/or health information about the patient's dependents and/or health information from other patients who have or had the covered disease. The probability value can also be determined based on health information from patients who have never had the covered disease.

The probability value can be determined using a trained machine learning model. The term "machine learning" refers to the development of algorithms and statistical models used by computer systems to perform a specific task without explicit instructions, relying instead on patterns and inference. Machine learning is seen as a subset of artificial intelligence. Machine learning algorithms build a mathematical model based on sample data, known as "training data", in order to make predictions or decisions without being explicitly programmed to perform the task.

The term "machine learning model", as used herein, may be understood as a computer implemented data processing architecture. The machine learning model can receive input data and provide output data based on that input data and on parameters of the machine learning model. The machine learning model can learn a relation between input data and output data through training. In training, parameters of the machine learning model may be adjusted in order to provide a desired output for a given input.

The process of training a machine learning model involves providing a machine learning algorithm (that is the learning algorithm) with training data to learn from. The term "trained machine learning model" refers to the model artifact that is created by the training process. The training data must contain the correct answer, which is referred to as the "target". The learning algorithm finds patterns in the training data that map input data to the target, and it outputs a trained machine learning model that captures these patterns.

In the training process, training data are inputted into the machine learning model and the machine learning model generates an output. The output is compared with the (known) target. Parameters of the machine learning model are modified in order to reduce the deviations between the output and the (known) target to a (defined) minimum.

A loss function may quantify the deviations between the output and the target. The loss function may be chosen in such a way that it rewards a wanted relation between output and target and/or penalizes an unwanted relation between an output and a target. Such a relation can be, e.g., a similarity, or a dissimilarity, or another relation.

If, for example, the output and the target are numbers, the loss function may be the difference between these numbers. In this case, a high absolute value of the loss function can mean that a parameter of the model needs to undergo a strong change.

In the case of vector-valued outputs, for example, difference metrics between vectors such as the root mean square error, a cosine distance, a norm of the difference vector such as a Euclidean distance, a Chebyshev distance, an Lp-norm of a difference vector, a weighted norm or any other type of difference metric of two vectors can be chosen. These two vectors may for example be the desired output (target) and the actual output.

In the case of higher dimensional outputs, such as two-dimensional, three-dimensional or higherdimensional outputs, for example an element-wise difference metric can be used. Alternatively or additionally, the output data may be transformed, for example to a one-dimensional vector, before computing a loss.

The modification of model parameters and the reduction of the loss can be done in an optimization procedure, for example gradient descent optimization, particle swarm optimization, genetic algorithm optimization, Levenberg-Marquardt optimization and/or other optimization procedure.

The machine learning model can be trained using training data from a plurality of reference patients.

The term "plurality" as it is used herein means an integer greater than 1, usually greater than 10, preferably greater than 100.

The term "reference" is used in this disclosure to distinguish the data used to train and/or validate the machine learning model from the data used to make predictions using the trained machine learning model. Thus, the data used to train and/or validate the machine learning model represent "reference patients" whereas data used for prediction purposes represent a new patient (i.e., a patient which is not a reference patient). However, the term "reference" is not to be understood in any other restrictive sense; this distinction serves only to prevent a clarity objection in patent grant proceedings.

The training data includes, for each reference patient among the plurality of reference patients, (i) health data of the reference patient as input data, and (ii) a disease information as target data.

The health data of the reference patient are data on the health status and/or lifestyle of the reference patient. They usually include the same data as the initial patient data.

The disease information indicates whether and/or at what time the reference patient has developed the covered disease and/or how severe the patient has had the covered disease.

Training the machine learning model usually involves the following steps:
for each reference patient:
- inputting the health data into the machine learning model,
- receiving a probability value as an output of the machine learning model,
- quantifying a deviation between the probability value and the disease information,
- reducing the deviation by modifying parameters of the machine learning model.

A loss function can be used to quantify the deviation between the probability value and the disease information. In an optimization procedure (e.g., a gradient descent procedure), such a deviation can be reduced by adjusting parameters of the machine learning model.

The training can be terminated if one or more stop criteria are met. Such stop criteria can be for example: a predefined maximum number of training steps has been performed, the deviations can no longer be reduced by modifying model parameters, a predefined minimum of the loss function is reached, and/or an extreme value of another performance value is reached.

As is common in training machine learning models, a cross-validation method (e.g., a 5-fold cross validation) can be used to split the training data into a training dataset and a holdout dataset. The training dataset is used for training of the model. The holdout dataset is used to verify that the trained model generalizes and makes good predictions.

It is also possible that instead of or in addition to a point prediction (the prediction of individual values) a probability distribution is predicted. In other words: the machine learning model can be a probabilistic prediction model.

In point prediction, the predictions are compared with the observed data (target) with a loss function. The analog in probabilistic regression may be a scoring rule that compares the estimated probability distribution with the observed data.

A proper scoring rule takes as input a predicted probability distribution and one observation (outcome) and assigns a score to the prediction such that the true distribution of the outcomes gets the best score in expectation. An optimization procedure such as a gradient descent approach can be used to find the model parameters that minimize the scoring rule. Preferably, the natural gradient is calculated, i.e., the direction of the steepest descent in Riemannian space.

However, it is also possible to use an ensemble of point predictors to predict a probability distribution.

Determining how likely a predicted value is has the advantage of knowing the uncertainty associated with the predicted value. The more uncertain a predicted value is, the greater the risk that it will not occur. An increased risk may be reflected in a higher price for a convertible bond and/or a shorter conversion period.

The trained machine learning model may be stored in a data memory and/or transmitted to a separate computer system and/or used to predict the likelihood of another patient to develop the covered disease.

The machine learning model may be a classification model, also called a classifier. The machine learning model may be configured to assign a (reference) patient to one of at least two classes based on health data (and optionally other data).

At least one class may include (reference) patients for whom the covered disease will erupt (or has erupted) within a specified time period (e.g., the conversion period), and one class may include (reference) patients for whom the disease will not erupt (has not erupted) within the specified time period. The probability value can indicate the probability with which a patient is assigned to a class.

There may be other classes, e.g., classes indicating how severe a (reference) patient will become (has become) ill with the covered disease.

The machine learning model can also be configured as a regression model that determines, e.g., the time period in which the covered disease occurs and/or the severity of the covered disease.

The probability value can be output, i.e., displayed on a monitor and/or output on a printer and/or stored in a data memory and/or transmitted to a separate computer system.

It is possible that the determined probability value is compared with a threshold value. If the probability value is below the threshold, the patient may be issued a convertible bond; if the probability value is equal to or above the threshold, the patient may be denied a convertible bond. Other scenarios are conceivable.

The probability value can be the basis for the calculation of further parameters, e.g., conditions of the convertible bond.

For example, based on the probability value, a price for the convertible bond and/or the duration of the conversion period and/or an interest rate at which interest is paid on the convertible bond may be determined.

It may be that the higher the probability that the patient will develop the covered disease and/or the earlier the covered disease is expected to occur and/or the more severe the expected course of the covered disease will be, the more expensive the convertible bond and/or the smaller the interest and/or the shorter the term of the convertible bond.

When the conditions on which the patient can obtain the convertible bond are communicated to the patient, the patient can decide whether to agree to the conditions and purchase a convertible bond.

If the patient agrees to the conditions, a contract is concluded between the distributor of the convertible bond or the therapy provider and the patient. The patient can give his or her consent to the conditions, e.g., by means of a digital signature.

It may be that the patient has to pay a one-time amount for the convertible bond, but it is also possible that the patient has to pay several amounts, which can be spread over the conversion period, for example.

Payments may be processed through the computer system of the present disclosure or through a separate payment processing service.

If the conversion criteria are met, the patient can convert the convertible bond into therapy.

Typically, the patient sends a request to convert the convertible bond to therapy to the distributor of the convertible bond or the provider of the therapy (this may depend on who the patient has contracted with for the convertible bond).

Such a request is received by the computer system of the present disclosure. Such a request can be submitted by the patient via email or through a graphical user interface over the Internet. However, such a request may also be received in writing by the convertible bond distributor or therapy provider and a user of the computer system of the present disclosure may enter such request into the computer system.

Such a request includes subsequent patient data. The subsequent patient data is used to check whether the conversion criteria are met.

The conversion criteria may be criteria that indicate the presence of the disease.

The conversion criteria may have been set by the distributor of the convertible bond and/or the therapy provider and/or an independent third party.

Such an independent third party may be, for example, a health authority or a health insurance company and/or a physician and/or a scientific institution and/or a medical institution.

It is also possible that the existence of the conversion criteria is verified by such an independent third party. It is possible that the patient or the convertible distributor or the therapy provider will forward the patient's request to the independent third party for review.

The check whether the conversion criteria are met is preferably performed automatically, i.e., without human intervention.

When checking whether the conversion criteria have been met, it is also possible to examine whether the conversion period has expired.

To determine if the conversion criteria are met, it is usually necessary to determine if the patient has the covered disease.

This examination may be performed by a physician who may communicate the examination results to the patient and/or the convertible bond distributor and/or the therapy provider. It is also possible that subsequent patient data will be transmitted, which will be analysed in an automated manner to detect signs of the covered disease in the subsequent patient data. The subsequent patient data may contain the same data as the initial patient data, except that it may have been collected at a later date (usually within the conversion period and before the patient has applied for conversion).

The initial patient data can also be used to check whether the patient has the covered disease. Differences between the initial patient data and the subsequent patient data can be detected. Such differences may indicate the presence of the covered disease.

A machine learning model or multiple machine learning models may be used to determine if the conversion criteria are met. There are numerous machine learning models that can be used to diagnose diseases.

If the conversion criteria are met, the therapy is released to the patient, i.e., the patient receives the therapy. If the conversion criteria are not met, the patient's request is rejected, i.e., the patient cannot exchange the convertible bond for the therapy; the patient does not receive the therapy.

It may be that the cost of the therapy is already covered by the purchase of the convertible bond, meaning that the patient may not have to pay an additional amount for the therapy; however, it is also possible that the patient will have to pay an amount for the therapy in addition to the amount of the convertible bond.

If the conversion period expires without the patient having submitted a request to exchange the convertible bond for therapy, the patient's right to convert expires.

Depending on the conditions agreed upon, the patient may receive a monetary amount and/or interest back at the end of the conversion period.

The computer system of this disclosure is configured to monitor all issued convertible bonds and nonissued convertible bonds. The computer system of the present disclosure is configured to record rejected applications for the issuance of convertible bonds, approved applications for the issuance of convertible bonds, rejected applications for the conversion of convertible bonds and approved applications for the conversion of convertible bonds, as well as the respective reasons that led to the rejection/approval.

This data may be used to adjust the conditions for the issuance and/or conversion of convertible bonds in the future.

The present invention is explained in more detail below with reference to the drawings, without intending to limit the invention to the features and/or combinations of features shown in the drawings.

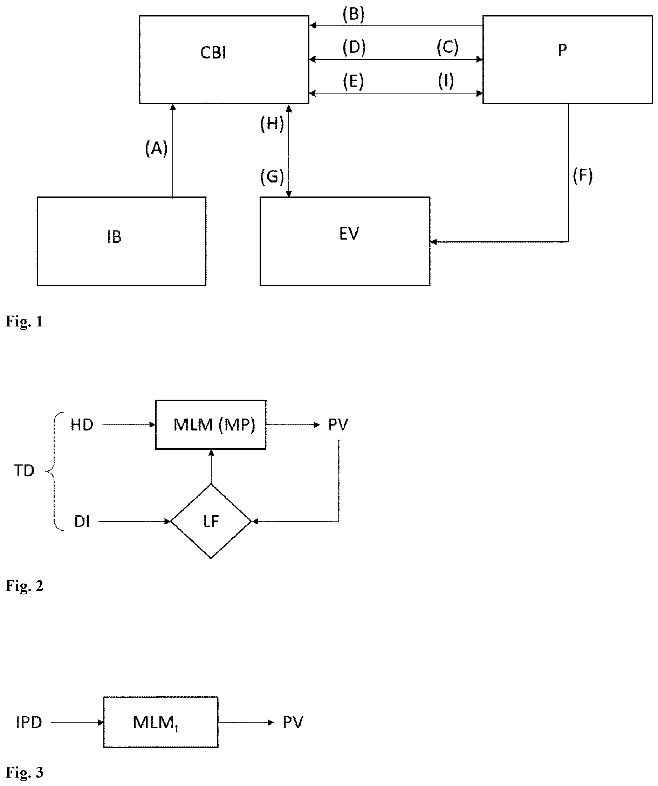

Fig. 1 schematically shows an example of an operating model for issuing convertible bonds and converting them into therapies. In Fig. 1, the arrows indicate transfers between participants in the operating model.

The operating model is designed to ensure (i) that patients are selected for therapies when they qualify and (ii) that those therapies are funded.

The issuer of the convertible bonds CBI is at the centre of the operating model. It may be the distributor of the convertible bonds and/or the provider of the therapy.

The convertible bonds issuer CBI develops and structures convertible bonds for therapies. An investment bank IB can provide financial support (A) if needed. Such an investment bank IB is optional.

The patient P submits an application for the issuance of a convertible bond and provides the information needed for the convertible bond issuer CBI to consider the application (B).

Whether patient P meets the prerequisites for issuing a convertible bond can be determined using a trained machine learning model, as described herein. If patient P satisfies the prerequisites, a convertible bond can be issued (C). The conditions for issuing the convertible bond can be determined based on the information provided by the patient P. The conditions can be determined using a trained machine learning model.

The patient pays a price for the issuance of the convertible bond (D).

It is possible that the evaluation of whether and/or on what terms to issue a convertible bond to the patient P is performed by an evaluator EV. The evaluator EV is usually an independent third party. Such an evaluator EV is optional. The evaluator EV can be a physician, for example. There can also be more than one evaluator.

If the patient P becomes ill with the disease covered by the convertible bond issued, the patient P applies for conversion of the convertible bond into therapy (E).

An evaluation is made as to whether the requirements for conversion of the convertible bond have been met. This evaluation can be carried out by the convertible bond issuer CBI and/or by the evaluator EV.

If an evaluator EV is involved in the evaluation, the patient may provide the required evaluation information directly to the evaluator EV (F) or the evaluator EV may obtain the information through the convertible bond issuer CBI (G).

If an evaluator EV is involved in the evaluation, it will submit the result of the evaluation to the convertible bond issuer CBI (H). In case the conditions for the conversion of the convertible bond into the therapy are met, the convertible bond issuer CBI releases the therapy for the patient P (I).

The patient P does not need to pay for the therapy, or the patient P has to pay an amount that is, however, less than the cost of the therapy. In addition, the price that the patient P has paid for the convertible bond is less than the cost of the therapy. Therefore, many patients can afford therapy.

Therapies may be financed by convertible bonds that have expired and have not been converted.

It is possible that, in the case of non-converted convertible bonds, (i) a portion of the price paid, (ii) the entire price paid, or (iii) the entire price paid, including interest, may be returned to the patient P. This depends, among other things, on the cost of therapy, the demand for convertible bonds and/or the amount of conversions of convertible bonds into therapies.

The issuer of the convertible bond CBI may use the proceeds from the sale of the convertible bonds and earn money from the spread, much like a bank. For example, if the convertible bond issuer CBI pays 1% interest, it may use the proceeds from the convertible bonds sale to pay off higher-interest debt or, ideally, invests in projects that promise a higher return. The issuer of the convertible bond CBI may use the proceeds for the development of therapies for diseases.

Figures 2 and 3 show schematically and by way of example the training of a machine learning model (Fig. 2) and the use of the trained machine learning model (Fig. 3) for assessing whether and/or on what conditions a convertible bond for a therapy is issued to a patent.

Fig. 2 shows the training procedure. The machine learning model MLM is trained based on training data TD. The training data TD contains (i) health data HD and a (ii) disease information DI for each reference patient among a plurality of reference patients.

In Fig. 2, only one training data set of one reference patient is shown; usually, there are several such training data sets.

The health data HD is information on the health status and/or lifestyle of the reference patient. It may also include health data of relatives of the reference patient.

The health data HD serve as input data for the machine learning model MLM.

The disease information DI is information about whether and/or at what time and/or how severely the reference patient is/was ill with the covered disease. The disease information DI serves as target data when training the machine learning model.

Thus, the machine learning model MLM is trained to learn a correlation between health data and information about whether/when/how severely a reference patient becomes ill with the covered disease.

A machine learning model usually requires numbers as input. The health data HD represents the health status and/or lifestyle of the reference patient in numerical form. The health data HD can be in the form of one or more feature vectors.

The term "vector" used in this disclosure also includes numbers, lists of numbers, vectors, matrices, tensors, and/or other arrangements of numbers.

Blood values and/or urine values and/or age and/or weight and/or height are features that are in numerical form and can be directly included in one or more feature vectors. Categorical variables such as gender, blood type, information about whether a patient smokes or does not smoke can be put into numerical form by arbitrarily assigning a number to each category. It is also possible to use one-hot encoding to convert categorical variables into a numerical representation.

Medical images can be transformed into feature vectors using a convolutional neural network and/or a vision transformer, for example.

Genetic information can be translated into feature vectors using pre-trained DNA language models, for example.

Different feature vectors can be fed to the machine learning model MLM through different inputs. However, it is also possible to combine different feature vectors into a joint feature vector before feeding the joint feature vector to the machine learning model. The combination of feature vectors can also be done in the machine learning model.

Combination(s) of (e.g., at least two) vectors to generate a joint vector may for example be achieved by a concatenation, a multiplication, an addition, for example an element-wise addition, a cross-product, and many other procedures. Preferred approaches for combining multimodal representations are described in: WO2023011943A1, WO2022228958A1, WO2022184516A1, WO2022106302A1.

The health data HD is input to the machine learning model MLM. The machine learning model MLM is configured to determine a probability value PV based on model parameters MP and based on the health data HD (and optionally other data). The probability value PV can be compared to the disease information DI. Deviations between the probability value PV and the disease information DI can be quantified with a loss function LF. The deviations can be reduced in an optimization procedure by modifying the model parameters MP.

The process described is performed on a variety of health data and disease information from a variety of reference patients until a stop criterion is met.

Such stop criteria can be for example: a predefined maximum number of training steps has been performed, the deviations can no longer be reduced by modifying model parameters, a predefined minimum of the loss function is reached, and/or an extreme value of another performance value is reached.

The trained machine learning model can be output, stored, transferred to a separate computer system, or used to determine a probability value for a new patient.

Fig. 3 shows the use of the trained machine learning model to determine a probability value for a new patient.

The new patient is a patient who has made a request to issue a convertible bond. Part of the request is initial patient data IPD. The initial patient data IPD is input to the trained machine learning model MLMₜ.

The trained machine learning model MLMt may have been trained as described with reference to Fig. 2. The trained machine learning model MLMₜ is configured and trained to determine a probability value PV based on the initial patient data IPD (and optionally other data).

The determined probability value PV may be output and/or stored and/or transmitted to a separate computer system.

The probability value PV can indicate the probability that the patient will develop the covered disease at all and/or within the conversion period. However, the probability value may also have another meaning as described in this disclosure.

The machine learning model may be or comprise an artificial neural network. The machine learning model may be or comprise a decision tree or a plurality of decision trees (e.g., a random forest). The machine learning model may be or include a support vector machine, a Naive Bayes classifier, and/or logistic regression.

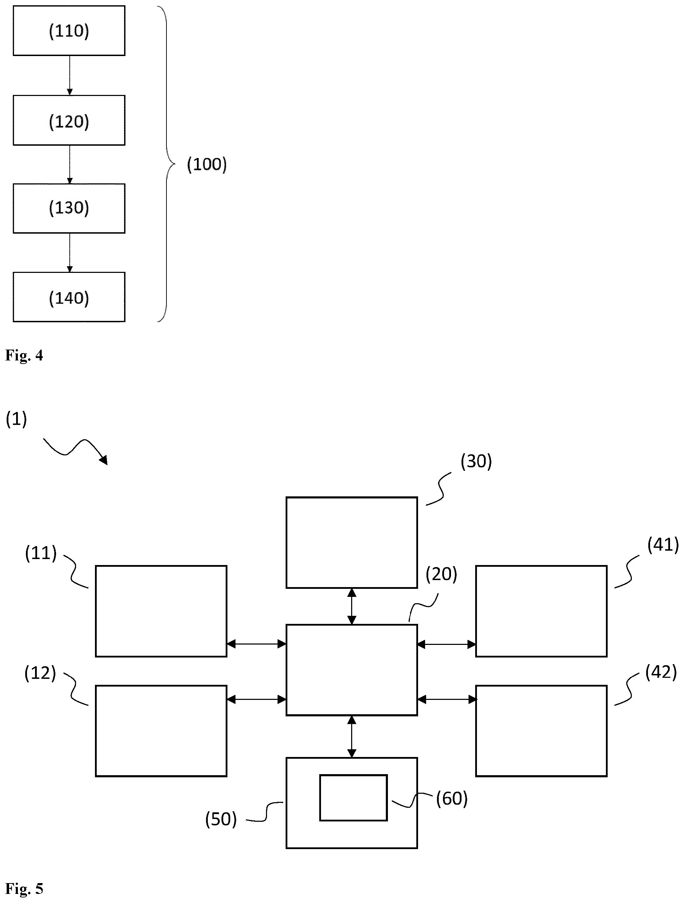

Fig. 4 shows schematically an embodiment of the computer-implemented method of the present disclosure in form of a flow chart.

The method (100) comprises the steps:
(110) receiving a request for the issuance of a convertible bond,
   ∘ wherein the convertible bond comprises a patient's right to receive a therapy for a disease if conversion criteria are met,
   ∘ wherein the request comprises initial patient data,
(120) determining whether issue criteria for issuance of the convertible bond to the patient are met based on the initial patient data,
(130) in the event that the issue criteria are met: determining the conditions under which the convertible bond is issued based on the initial patient data and/or based on data on convertible bonds already issued to other patients and/or based on data from other patients who suffered from the disease,
(140) outputting the conditions under which the convertible bond is issued.

The operations in accordance with the teachings herein may be performed by at least one computer system specially constructed for the desired purposes or general-purpose computer system specially configured for the desired purpose by at least one computer program stored in a typically non-transitory computer readable storage medium.

A "computer system" is a system for electronic data processing that processes data by means of programmable calculation rules. Such a system usually comprises a "computer", that unit which comprises a processor for carrying out logical operations, and also peripherals.

In computer technology, "peripherals" refer to all devices which are connected to the computer and serve for the control of the computer and/or as input and output devices. Examples thereof are monitor (screen), printer, scanner, mouse, keyboard, drives, camera, microphone, loudspeaker, etc. Internal ports and expansion cards are, too, considered to be peripherals in computer technology.

Computer systems of today are frequently divided into desktop PCs, portable PCs, laptops, notebooks, netbooks and tablet PCs and so-called handhelds (e.g. smartphone); all these systems can be utilized for carrying out the invention.

The term "non-transitory" is used herein to exclude transitory, propagating signals or waves, but to otherwise include any volatile or non-volatile computer memory technology suitable to the application.

The term "computer" should be broadly construed to cover any kind of electronic device with data processing capabilities, including, by way of non-limiting example, personal computers, servers, embedded cores, computing system, communication devices, processors (e.g., digital signal processor (DSP)), microcontrollers, field programmable gate array (FPGA), application specific integrated circuit (ASIC), etc.) and other electronic computing devices.

The term "process" as used above is intended to include any type of computation or manipulation or transformation of data represented as physical, e.g., electronic, phenomena which may occur or reside e.g., within registers and/or memories of at least one computer or processor. The term processor includes a single processing unit or a plurality of distributed or remote such units.

Fig. 5 illustrates a computer system (1) according to some example implementations of the present disclosure in more detail.

Generally, a computer system of exemplary implementations of the present disclosure may be referred to as a computer and may comprise, include, or be embodied in one or more fixed or portable electronic devices. The computer may include one or more of each of a number of components such as, for example, a processing unit (20) connected to a memory (50) (e.g., storage device).

The processing unit (20) may be composed of one or more processors alone or in combination with one or more memories. The processing unit (20) is generally any piece of computer hardware that is capable of processing information such as, for example, data, computer programs and/or other suitable electronic information. The processing unit (20) is composed of a collection of electronic circuits some of which may be packaged as an integrated circuit or multiple interconnected integrated circuits (an integrated circuit at times more commonly referred to as a "chip"). The processing unit (20) may be configured to execute computer programs, which may be stored onboard the processing unit (20) or otherwise stored in the memory (50) of the same or another computer.

The processing unit (20) may be a number of processors, a multi-core processor or some other type of processor, depending on the particular implementation. For example, it may be a central processing unit (CPU), a field programmable gate array (FPGA), a graphics processing unit (GPU) and/or a tensor processing unit (TPU). Further, the processing unit (20) may be implemented using a number of heterogeneous processor systems in which a main processor is present with one or more secondary processors on a single chip. As another illustrative example, the processing unit (20) may be a symmetric multi-processor system containing multiple processors of the same type. In yet another example, the processing unit (20) may be embodied as or otherwise include one or more ASICs, FPGAs or the like. Thus, although the processing unit (20) may be capable of executing a computer program to perform one or more functions, the processing unit (20) of various examples may be capable of performing one or more functions without the aid of a computer program. In either instance, the processing unit (20) may be appropriately programmed to perform functions or operations according to example implementations of the present disclosure.

The memory (50) is generally any piece of computer hardware that is capable of storing information such as, for example, data, computer programs (e.g., computer-readable program code (60)) and/or other suitable information either on a temporary basis and/or a permanent basis. The memory (50) may include volatile and/or non-volatile memory, and may be fixed or removable. Examples of suitable memory include random access memory (RAM), read-only memory (ROM), a hard drive, a flash memory, a thumb drive, a removable computer diskette, an optical disk, a magnetic tape or some combination of the above. Optical disks may include compact disk - read only memory (CD-ROM), compact disk - read/write (CD-R/W), DVD, Blu-ray disk or the like. In various instances, the memory may be referred to as a computer-readable storage medium or data memory. The computer-readable storage medium is a non-transitory device capable of storing information, and is distinguishable from computer-readable transmission media such as electronic transitory signals capable of carrying information from one location to another. Computer-readable medium as described herein may generally refer to a computer-readable storage medium or computer-readable transmission medium.

In addition to the memory (50), the processing unit (20) may also be connected to one or more interfaces for displaying, transmitting and/or receiving information. The interfaces may include one or more communications interfaces and/or one or more user interfaces. The communications interface(s) may be configured to transmit and/or receive information, such as to and/or from other computer(s), network(s), database(s) or the like. The communications interface may be configured to transmit and/or receive information by physical (wired) and/or wireless communications links. The communications interface(s) may include interface(s) (41) to connect to a network, such as using technologies such as cellular telephone, Wi-Fi, satellite, cable, digital subscriber line (DSL), fiber optics and the like. In some examples, the communications interface(s) may include one or more short-range communications interfaces (42) configured to connect devices using short-range communications technologies such as NFC, RFID, Bluetooth, Bluetooth LE, ZigBee, infrared (e.g., IrDA) or the like.

The user interfaces may include a display (30). The display (screen) may be configured to present or otherwise display information to a user, suitable examples of which include a liquid crystal display (LCD), light-emitting diode display (LED), plasma display panel (PDP) or the like. The user input interface(s) (11) may be wired or wireless, and may be configured to receive information from a user into the computer system (1), such as for processing, storage and/or display. Suitable examples of user input interfaces include a microphone, image or video capture device, keyboard or keypad, joystick, touch-sensitive surface (separate from or integrated into a touchscreen) or the like. In some examples, the user interfaces may include automatic identification and data capture (AIDC) technology (12) for machine-readable information. This may include barcode, radio frequency identification (RFID), magnetic stripes, optical character recognition (OCR), integrated circuit card (ICC), and the like. The user interfaces may further include one or more interfaces for communicating with peripherals such as printers and the like.

As indicated above, program code instructions (60) may be stored in memory (50), and executed by processing unit (20) that is thereby programmed, to implement functions of the systems, subsystems, tools and their respective elements described herein. As will be appreciated, any suitable program code instructions (60) may be loaded onto a computer or other programmable apparatus from a computer-readable storage medium to produce a particular machine, such that the particular machine becomes a means for implementing the functions specified herein. These program code instructions (60) may also be stored in a computer-readable storage medium that can direct a computer, processing unit or other programmable apparatus to function in a particular manner to thereby generate a particular machine or particular article of manufacture. The instructions stored in the computer-readable storage medium may produce an article of manufacture, where the article of manufacture becomes a means for implementing functions described herein. The program code instructions (60) may be retrieved from a computer-readable storage medium and loaded into a computer, processing unit or other programmable apparatus to configure the computer, processing unit or other programmable apparatus to execute operations to be performed on or by the computer, processing unit or other programmable apparatus.

Retrieval, loading and execution of the program code instructions (60) may be performed sequentially such that one instruction is retrieved, loaded and executed at a time. In some example implementations, retrieval, loading and/or execution may be performed in parallel such that multiple instructions are retrieved, loaded, and/or executed together. Execution of the program code instructions (60) may produce a computer-implemented process such that the instructions executed by the computer, processing circuitry or other programmable apparatus provide operations for implementing functions described herein.

Execution of instructions by processing unit, or storage of instructions in a computer-readable storage medium, supports combinations of operations for performing the specified functions. In this manner, a computer system (1) may include processing unit (20) and a computer-readable storage medium or memory (50) coupled to the processing circuitry, where the processing circuitry is configured to execute computer-readable program code instructions (60) stored in the memory (50). It will also be understood that one or more functions, and combinations of functions, may be implemented by special purpose hardware-based computer systems and/or processing circuitry which perform the specified functions, or combinations of special purpose hardware and program code instructions.

The computer system of the present disclosure may be in the form of a laptop, notebook, netbook, and/or tablet PC.

## Claims

1. A computer-implemented method for selecting a patient for a therapy, the method comprising:
- receiving a request for the issuance of a convertible bond,
∘ wherein the convertible bond comprises a patient's right to receive a therapy for a disease if conversion criteria are met,
∘ wherein the request comprises initial patient data,
- determining whether issue criteria for issuance of the convertible bond to the patient are met based on the initial patient data,
- in the event that the issue criteria are met: determining the conditions under which the convertible bond is issued based on the initial patient data and/or based on data on convertible bonds already issued to other patients and/or based on data from other patients who suffered from the disease,
- outputting the conditions under which the convertible bond is issued.

2. The method of claim 1, further comprising:
- receiving a request for conversion of the convertible bond, the request comprising subsequent patient data,
- determining whether the conversion criteria are met,
- in case the conversion conditions are met: notifying the patient and/or a physician that the patient will receive therapy.

3. The method of claim 1 or 2, wherein the therapy is an individualized therapy adapted to the individual needs of the patient.

4. The method of any one of claims 1 to 3, wherein the therapy is or comprises gene therapy, cell therapy and/or a therapy for treating a rare disease.

5. The method of any one of claims 1 to 4, wherein determining whether the issue criteria are met includes:
- determining whether the patient already has the disease, and/or
- determining whether one or more of patient's dependents have or had the disease.

6. The method of any one of claims 1 to 5, wherein determining whether the issue criteria are met includes:
- determining a probability value, the probability value indicating a probability that the patient will develop the disease at all and/or within a pre-defined time period.

7. The method of any one of claims 1 to 6, wherein determining whether the conversion criteria are met includes:
- determining if the patient has the disease, and/or
- determining whether a conversion period has expired.

8. The method of any one of claims 1 to 7, wherein determining whether the conversion criteria are met includes:
- determining a probability value, the probability value indicating a probability that the patient will develop the disease at all and/or within a pre-defined time period.

9. The method of any one of claims 6 to 8, wherein the probability value is determined using a trained machine learning model.

10. The method of claim 9, wherein the trained machine learning model is configured and was trained to determine a probability value based on the initial patient data, wherein the trained machine learning model was trained on training data, wherein the training data comprised, for each reference patient of a plurality of reference patients, (i) health data of the reference patient as input data and (ii) a disease information as target data, the disease information indicating whether and/or when the reference patient had the disease.

11. The method of any one of claims 1 to 10, wherein initial patient data includes one or more of the following: patient's age, sex, body size, body weight, body mass index, blood group, blood values, blood pressure values, resting heart rate, heart rate variability, an electrocardiogram, sugar concentration in urine and/or blood and/or other values from urine/blood tests, body temperature, impedance, lifestyle information about the life of the patient, including consumption of alcohol, smoking, and/or exercise and/or the patient's diet, medical intervention parameters including regular medication, occasional medication, and/or other previous or current medical interventions and/or other information about the patient's previous and/or current treatments and/or reported health conditions, results of genetic testing on the patient and/or other results of other laboratory tests, one or more medical images including x-rays and/or magnetic resonance imaging images and/or computed tomography images and/or ultrasound images and/or positron emission tomography images and/or combinations thereof.

12. A computer system comprising:
a processor; and
a memory storing an application program configured to perform, when executed by the processor, an operation, the operation comprising:
- receiving a request for the issuance of a convertible bond,
∘ wherein the convertible bond comprises a patient's right to receive a therapy for a disease if conversion criteria are met,
∘ wherein the request comprises initial patient data,
- determining whether issue criteria for issuance of the convertible bond to the patient are met based on the initial patient data,
- in the event that the issue criteria are met: determining the conditions under which the convertible bond is issued based on the initial patient data and/or based on data on convertible bonds already issued to other patients and/or based on data from other patients who suffered from the disease,
- outputting the conditions under which the convertible is issued.

13. A non-transitory computer readable storage medium having stored thereon software instructions that, when executed by a processor of a computer system, cause the computer system to execute the following steps:
- receiving a request for the issuance of a convertible bond,
∘ wherein the convertible bond comprises a patient's right to receive a therapy for a disease if conversion criteria are met,
∘ wherein the request comprises initial patient data,
- determining whether issue criteria for issuance of the convertible bond to the patient are met based on the initial patient data,
- in the event that the issue criteria are met: determining the conditions under which the convertible bond is issued based on the initial patient data and/or based on data on convertible bonds already issued to other patients and/or based on data from other patients who suffered from the disease,
- outputting the conditions under which the convertible is issued.
